# EUROPEAN PATENT APPLICATION

(11) **EP 4 843 998 A1**
(43) Date of publication of application: **25.11.2026**
(21) Application number: 25177580.5
(22) Date of filing: 20.05.2025
(51) Int. Cl.: C07K 14/475, C12N 9/50

(54) **RECOMBINANT HUMAN BRAIN-DERIVED NEUROTROPHIC FACTOR, AND METHOD OF PRODUCTION THEREOF**

(71) Applicant: Dompé farmaceutici SpA, 20122 Milano (IT)
(72) Inventor: Martin, Franck, L'Aquila (IT); Spaccapaniccia, Elisa, L'Aquila (IT); Maffei, Mariano, L'Aquila (IT); Antonosante, Andrea, L'Aquila (IT); De Simone, Lucio, L'Aquila (IT); Cazzorla, Tiziano, L'Aquila (IT); Bertozzi, Serena, L'Aquila (IT)
(74) Representative: Dompé farmaceutici Spa

(57) **Abstract**

The present invention relates to a human pro-BDNF mutein and to a method of recombinantly producing a correctly folded, biologically active human brain-derived neurotrophic factor (BDNF).

## Description

### FIELD OF THE INVENTION

The present invention relates to a human pro-BDNF mutein and to a method of recombinantly producing it in a prokaryotic system.

### STATE OF THE ART

Neurotrophins are a family of growth factors crucial for the regulation of neuronal maintenance, differentiation, and survival. In particular, neurotrophin signaling regulates a diverse set of neural processes including differentiation, neurite outgrowth, axon pruning, apoptosis, and cell survival during development, adulthood, and following injury to the nervous system.

Neurotrophins mediate these actions by binding different receptors: tropomyosin-related kinase (Trk) receptor tyrosine kinases, the p75 neurotrophin receptor, and sortilin family members. The human neurotrophin family is composed of nerve growth factor (NGF), brain-derived neurotrophic factor (BDNF), neurotrophin-3 (NT-3) and neurotrophin-4 (NT-4). Each of these structurally related neurotrophins is synthesized as a precursor prior to proteolytic cleavage to produce a mature neurotrophin (Huang E.J. et al., Annu. Rev. Biochem. 2003; 72: 609-642).

Mature human BDNF is a cystine-knot protein, having 119 amino acid residues **(SEQ ID NO:1**, Gray K. et al., FEBS letters. 2008; 582 (6): 907-10), which represents the functional domain of the protein, where three disulphide bonds are located (Robinson R.C. et al., Protein Science. 1999;8(12):2589-97). The protein gene of BDNF is on 11 p human chromosome (Tian F. et al, Amino acids. 2010;38(4):1067-74).

BDNF binds at least two cell surface receptors, TrkB and p75, that can respond to this growth factor. This signaling molecule has been well-documented for its ability to regulate neuronal plasticity, cell growth, proliferation, cell survival, and long-term memory. In particular, it has been shown to act on certain neurons of the central nervous system and the peripheral nervous system, helping to support survival of existing neurons, and encouraging growth and differentiation of new neurons and synapses. In the brain it is active in the hippocampus, cortex, and basal forebrain-areas vital to learning, memory, and higher thinking. BDNF is also expressed in the retina, kidneys, prostate, motor neurons, and skeletal muscles, and is also found in saliva.

Currently, to be administered as a therapeutic molecule, BDNF is recombinantly produced.

To be clinically developed, recombinant human BDNF (rhBDNF) must meet current regulations regarding the production of clinically acceptable therapeutic products. In particular, it is necessary to produce recombinant human BDNF that is characterized by a sufficiently high degree of purity, a consistent composition and activity profile.

An optimal recombinant human BDNF production method for therapeutic use must therefore ensure the production of a molecule satisfying these requirements and be suitable to be adapted to an industrial large-scale production process.

However, the industrial production process of rhBDNF has always been problematic.

In fact, in Eukaryotic expression systems, rhBDNF is produced at a very low yield, whereas in Prokaryotic expression systems, as for example in *Escherichia coli,* such a protein is typically accumulated in an unfolded and aggregated form as inclusion bodies; thus, a refolding step is needed to obtain the correctly-folded, biologically active rhBDNF.

In this regard (namely, in prokaryotic expression systems), it is known that rhBDNF poorly refolds without its pro-sequence chaperon effect; thus, the refolding step must be carried out on the pro-BDNF (and not on the mature BDNF form, which is free from the pro-sequence, also called as "pro-peptide"), to ensure that a correct folding takes place, thus preserving its structural cystine-knot (i.e., a protein structural motif containing three disulfide bridges).

However, once pro-BDNF is correctly refolded, the cleavage of said pro-sequence from pro-BDNF, to obtain a correctly folded mature rhBDNF, becomes the production process bottleneck.

Indeed, if the proteolytic enzyme is not highly specific for cleaving the peptide bond between the last pro-sequence amino acid and the first amino acid of the mature rhBDNF, undesired by-products of enzymatic digestion (hereinafter, "pro-BDNF digestion by-products") are obtained, for example hypo-digested and hyper-digested rhBDNF forms, which decrease yield and purity of the correctly folded, biologically active rhBDNF obtained during the process. Consequently, several purification steps are needed to try to obtain the highest possible yield and purity of said rhBDNF.

In this regard, EP 4 413 993 and WO 2024/165725 of the present Applicant disclose a specific human pro-BDNF mutein characterized by a mutated sequence in the pro-sequence, so that a trypsin protease is able to cleave the pro-sequence from pro-BDNF with a higher specificity as compared to the wild-type human pro-BDNF.

While said mutein being actually more efficient for the production of rhBDNF in respect of the wild-type pro-BDNF in a large-scale process, it has been noted that a certain amount of pro-BDNF digestion by-products are obtained, which generally include the following hypo-digested and hyper-digested rhBDNF forms:
**SEQ ID NO:2** (a hypo-digested rhBDNF form): 
GLTSLADTFE HVIEELLDED QKVRPNEENN KDADLYTSRV MLSSQVPLEP PLLFLLEEYK
NYLDAANMSM RVRRHSDPAR RGELSVCDTI SEWVTAADKK TAVDMSGGTV TVLEKVPVSK
GQLKQYFYET KCNPMGYTKE GCRGIDKRHW NSQCRTTQSY VRALTMDSKK RIGWRFIRID
TSCVCTLTIK RGR
**SEQ ID NO:3** (a hypo-digested rhBDNF form) 
VMLSSQVPLE PPLLFLLEEY KNYLDAANMS MRVRRHSDPA RRGELSVCDT ISEWVTAADK
KTAVDMSGGT VTVLEKVPVS KGQLKQYFYE TKCNPMGYTK EGCRGIDKRH WNSQCRTTQS
YVRALTMDSK KRIGWRFIRI DTSCVCTLTI KRGR
**SEQ ID NO:4** (a hypo-digested rhBDNF form) 
VRRHSDPARR GELSVCDTIS EWVTAADKKT AVDMSGGTVT VLEKVPVSKG QLKQYFYETK
CNPMGYTKEG CRGIDKRHWN SQCRTTQSYV RALTMDSKKR IGWRFIRIDT SCVCTLTIKR
GR
**SEQ ID NO:5** (a hypo-digested rhBDNF form) 
RHSDPARRGE LSVCDTISEW VTAADKKTAV DMSGGTVTVL EKVPVSKGQL KQYFYETKCN
PMGYTKEGCR GIDKRHWNSQ CRTTQSYVRA LTMDSKKRIG WRFIRIDTSC VCTLTIKRGR
**SEQ ID NO:6** (a hyper-digested rhBDNF form)

RGELSVCDTI SEWVTAADKK TAVDMSGGTV TVLEKVPVSK GQLKQYFYET KCNPMGYTKE
GCRGIDKRHW NSQCRTTQSY VRALTMDSKK RIGWRFIRID TSCVCTLTIK RGR

In particular, it has been noted that the rhBDNF forms of SEQ ID NO: 2 and SEQ ID NO: 3 are the main by-products of hypo-digested kind, and correspond each to 10-15 % wt. of the digested product, and that rhBDNF hyper-digested form of SEQ ID NO: 6 corresponds to 10-15 % wt. of the digested product, said digestion by-products being determined and calculated before the final step of purification, after which the final product is obtained and ready to be administered.

This occurs because the trypsin protease, as well as trypsin-like proteases, generally cleaves peptide bonds following an Arginine or a Lysine, and the human pro-BDNF comprises several of those amino acids in its sequence.

In the light of the above, it is felt the need to develop a new and improved human pro-BDNF mutein, and a process for the production of rhBDNF starting from said mutein, in order to further reduce the amount of the pro-BDNF digestion by-products and consequently optimize the production process on a large-scale basis.

### SUMMARY OF THE INVENTION

The present invention relates to a human pro-BDNF mutein comprising, preferably consisting of, the amino acid sequence set forth in **SEQ ID NO:8**, as reported below:

MAPMKEANIR GQGGLAYPGV RTHGTLESVN GPKAGSRGLT SLADTFEHVI EELLDEDQKV
RPNEENNKDA DLYTSRVMLS SQVPLEPPLL FLLEEYKNYL DAANMENLYF QHSDPARRGE
LSVCDSISEW VTAADKKTAV DMSGGTVTVL EKVPVSKGQL KQYFYETKCN PMGYTKEGCR
GIDKRHWNSQ CRTTQSYVRA LTMDSKKRIG WRFIRIDTSC VCTLTIKRGR

A further object of the present invention is a nucleic acid encoding for said human pro-BDNF mutein.

A further object of the present invention is a process of recombinantly producing a correctly folded, biologically active human BDNF (rhBDNF) from a pro-BDNF mutein, in a prokaryotic expression system, according to the present invention.

A further object of the present invention is a human BDNF obtained, or obtainable, by the method described above.

A further object of the present invention is the use of a pro-BDNF mutein according to the invention for the preparation of a correctly folded, biologically active rhBDNF.

### BRIEF DESCRIPTION OF THE FIGURES

**Figure 1** reports the results of the SDS-PAGE analysis of the products digested by wild-type TEV as disclosed in Example 1, and by Numacut^{™} TEV protease as disclosed in Example 2.
**Figure 2** shows a graph reporting the concentration-response (from 0.23 µg/mL to 9 µg/mL) proliferation curve of C6 cells incubated with a rhBDNF reference standard, and a rhBDNF obtained according to the present invention, as disclosed in Example 1.
**Figure 3** reports the results of the SDS-PAGE analysis of the products digested by an enterokinase protease, as disclosed in Example 3.
**Figure 4** reports the results of the SDS-PAGE analysis of the products digested by a Factor Xa protease, as disclosed in Example 3.
**Figure 5** reports the results of the SDS-PAGE analysis of the products digested by a furin protease, as disclosed in Example 3.

### DETAILED DESCRIPTION OF THE INVENTION

### Definitions

The expression "human wild type pro-BDNF" or "human pro-BDNF" refers to the precursor of human BDNF, having the amino acid sequence of **SEQ ID NO: 7:**

MAPMKEANIR GQGGLAYPGV RTHGTLESVN GPKAGSRGLT SLADTFEHVI EELLDEDQKV
RPNEENNKDA DLYTSRVMLS SQVPLEPPLL FLLEEYKNYL DAANMSMRVR RHSDPARRGE
LSVCDSISEW VTAADKKTAV DMSGGTVTVL EKVPVSKGQL KQYFYETKCN PMGYTKEGCR
GIDKRHWNSQ CRTTQSYVRA LTMDSKKRIG WRFIRIDTSC VCTLTIKRGR

Optionally, said human pro-BDNF may be a "recombinant human pro-BDNF" (rh-pro-BDNF), namely said "human pro-BDNF" is produced recombinantly.

The expression "human pro-BDNF mutein" or "human pro-BDNF mutant" refers to a human pro-BDNF containing one or more amino acids replacements; optionally, said human pro-BDNF mutant may be a "recombinant human pro-BDNF mutant" (rh-pro-BDNF mutant), namely said "human pro-BDNF mutant" is produced recombinantly.

After digestion of the pro-sequence of said pro-BDNF by a protease, a "mature" human BDNF is obtained.

The expression "human wild type BDNF" or "human BDNF" refer to the human form of the protein BDNF, having the amino acid sequence of **SEQ ID NO:1:**

HSDPARRGEL SVCDSISEWV TAADKKTAVD MSGGTVTVLE KVPVSKGQLK
QYFYETKCNP MGYTKEGCRG IDKRHWNSQC RTTQSYVRAL TMDSKKRIGW
RFIRIDTSCV CTLTIKRGR

Optionally, said human BDNF may be a "recombinant human BDNF" (rhBDNF), namely said "human BDNF" is produced recombinantly.

The expression "correctly folded" as used herein means:
- when referred to human wild type BDNF/human BDNF, a human wild type BDNF with a three-dimensional structure corresponding to that of the native biologically active human BDNF.
- when referred to human pro-BDNF mutein, human pro-BDNF mutein with a three-dimensional structure that results in the formation of a correctly folded human BDNF upon cleavage of the pro-sequence.

The expression "biologically active" referring to human BDNF or rhBDNF, means that said human BDNF or rhBDNF are characterized by a biological activity corresponding to that of the native wild-type biologically active human BDNF. Said activity may be, for example, determined according to as disclosed in Example 1.

Preferably, said biologically active human BDNF or rhBDNF is correctly folded, according to the definitions provided above.

The terms "non-folded", "unfolded" and "denatured" are herein synonyms.

### Description of the invention

As will be explained more in detail in the experimental section, the inventors have developed a method of recombinantly producing human brain-derived neurotrophic factor (rhBDNF), which is advantageous over the methods disclosed in the prior art, since it is simple, reduces the pro-BDNF digestion by-products, and allows to obtain a correctly folded, biologically active rhBDNF with high purity and high yields.

This is achieved by expressing a human pro-BDNF mutein as described below as a precursor of rhBDNF, and by carrying out the process as will be discussed below.

### - Human pro-BDNF mutein of the invention

According to an embodiment of the present invention, a human pro-BDNF mutein comprising, preferably consisting of, the amino acid sequence set forth in **SEQ ID NO:8** is provided.

In particular, in said mutein, the last six amino acids of the pro-sequence (P1-P6) of human pro-BDNF have been replaced with the TEV protease recognition site sequence amino acids, as reported in Table 1.

**Table 1: P1, P2, P3, P4, P5 and P6 are the last six amino acid positions (P) of the pro-sequence of the human pro-BDNF; "WT" means human wild-type pro-BDNF, "TEV" (in the "Clone" column) means the human pro-BDNF mutein, according to the present invention, comprising the recognition site sequence of a TEV protease.**

| **Clone** | **Enzyme** | **P6** | **P5** | **P4** | **P3** | **P2** | **P1** | |
|---|---|---|---|---|---|---|---|---|
| Pro-BDNF-WT | Furin | S | M | R | V | R | R | **SEQ ID NO:9** |
| Pro-BDNF-TEV | TEV | E | N | L | Y | F | Q | **SEQ ID NO:10** |

Advantageously, the amino acid sequence of SEQ ID NO:10 is the recognition site sequence (cleavage site) of a TEV protease, and it is a "single recognition site sequence" in the pro-BDNF mutein sequence according to the present invention, i.e. there are not any other sequence(s) of SEQ ID NO:10 in said pro-BDNF mutein.

Such a single recognition site of TEV protease in the human pro-BDNF mutein sequence, according to the present invention, advantageously allows to carry out an unpredicted highly efficient cleavage of the pro-sequence from said human pro-BDNF mutein, thus drastically reducing the formation of pro-BDNF digestion by-products (i.e. hypo- and/or hyper-digested human BDNF forms) in the process for the production of a correctly folded, biologically active human (preferably, recombinant human) BDNF.

The TEV protease is unexpectedly advantageous in respect to other proteases recognizing a single site, for efficiently cleaving the pro-sequence from the human pro-BDNF sequence according to the process of the present invention.

In fact, as shown in the Experimental section below, human pro-BDNF muteins have been modified to include the single recognition site sequence of furin, Factor Xa, and enterokinase, respectively, in replacement of the recognition site sequence of TEV, and they were used as the starting material for processes producing a correctly folded, biologically active rhBDNF.

However, each of said processes reported several disadvantages and failures, including mainly poor quality and yield of the final product (i.e. a correctly folded, biologically active rhBDNF).

Moreover, said single recognition site sequence of TEV protease does not advantageously perturb the correct refolding of the unfolded human pro-BDNF mutein according to the present invention, which is obtained by dissolving the inclusion bodies aggregated in a suitable expression system, preferably a prokaryotic expression system, in which said pro-BDNF has been previously expressed.

Thus, the replacement of the wild-type sequence of SEQ ID NO: 9 with said single recognition site sequence of TEV protease (SEQ ID NO:10) unexpectedly does not influence the correct refolding of the mutein according to the present invention, and it consequently guarantees the preservation of the characteristic structural cystine-knot of rhBDNF. Advantageously, such a replacement does not perturb any of the process steps according to the present invention, as discussed below.

The present invention is also directed to nucleic acids encoding for the pro-BDNF muteins described herein as well.

A preferred nucleic acid sequence of the pro-BDNF mutein according to the present invention comprises, preferably consists of, the sequence set forth in **SEQ ID NO:11:**

ATG GCG CCG ATG AAA GAG GCG AAC ATT CGC GGA CAA GGT GGC TTG GCC
TAT CCA GGC GTT CGT ACA CAT GGC ACC TTA GAA TCC GTG AAT GGT CCG
AAA GCT GGA AGT CGT GGG CTT ACC AGC TTA GCG GAC ACC TTT GAA CAC
GTG ATT GAA GAA CTG CTG GAT GAA GAT CAG AAA GTA CGG CCC AAT GAG
GAG AAC AAC AAA GAT GCG GAC CTC TAT ACG AGC CGT GTG ATG CTG TCA

AGT CAG GTT CCG TTA GAA CCG CCA CTG TTG TTC CTT CTG GAA GAA TAC
AAG AAC TAT CTG GAT GCA GCG AAC ATG GAA AAC CTG TAT TTT CAG CAT
TCC GAT CCT GCA CGT CGC GGT GAA CTG AGC GTG TGC GAT AGC ATC TCT
GAA TGG GTG ACC GCC GCC GAT AAG AAA ACG GCC GTT GAT ATG TCT GGT
GGT ACC GTA ACC GTT CTG GAG AAA GTC CCG GTA TCG AAA GGC CAG CTG
AAA CAG TAC TTC TAT GAG ACT AAG TGC AAT CCT ATG GGC TAC ACG AAA
GAA GGC TGT CGC GGC ATT GAC AAA CGG CAC TGG AAT TCG CAA TGT CGC
ACT ACG CAG TCC TAT GTC CGT GCA CTC ACT ATG GAC TCT AAG AAA CGC
ATT GGT TGG CGC TTT ATC CGC ATT GAC ACA TCG TGC GTC TGT ACC CTG
ACG ATC AAA CGT GGG CGC TAA TGA

### - Process for the production of BDNF according to the present invention

The present invention also relates to a process of recombinantly producing human BDNF (rhBDNF), preferably a correctly folded, biologically active human BDNF (rhBDNF), from a human pro-BDNF mutein according to present invention, in a prokaryotic expression system, as disclosed below.

Preferably, said process comprises the steps of:
a) providing a human pro-BDNF mutein, preferably a correctly folded human pro-BDNF mutein, according to the present invention;
b) cleaving the pro-sequence of said human pro-BDNF mutein by a TEV protease, thus obtaining said rhBDNF, preferably said correctly folded, biologically active rhBDNF; and
c) isolating and purifying said rhBDNF, preferably said correctly folded, biologically active rhBDNF.

### - Step a) of the process according to the present invention

The above-mentioned human pro-BDNF mutein is recombinantly produced in a prokaryotic expression system, as disclosed below.

In a preferred embodiment of the present invention, said step a) comprises a step i) of expressing a human pro-BDNF mutein according to the present invention in a prokaryotic expression system, more preferably in the form of inclusion bodies into said prokaryotic expression system.

Preferably, said prokaryotic expression system is a bacterial expression system, more preferably selected from the group comprising *E*. *coli, Bacillus sp.,* and *Salmonella;* even more preferably, it is *E*. *coli,* and most preferably it is selected from the group comprising *E*. *coli BL21 (DE3), JM 108*/*109 (K12), JM106, JM83* and *TBI.*

To transform said prokaryotic expression system, a nucleic acid sequence encoding said human pro-BDNF according to the present invention is introduced into a suitable expression vector, preferably a prokaryotic expression vector, even more preferably selected from pET28, pBR322, pMAL, pUC19 and all derivatives, most preferably pET28. However, all suitable expression vectors known in the art may be used for this purpose.

Preferably, said nucleic acid sequence encoding said human pro-BDNF mutein according to the present invention is DNA; more preferably, it is codon-optimized DNA sequence for an efficient expression in the prokaryotic expression system, most preferably, it a codon-optimized DNA sequence for the expression in *E*. *coli.*

Even more preferably, said nucleic acid sequence is set forth in SEQ ID NO: 11.

Said expression vector comprising the nucleic acid sequence encoding said human pro-BDNF according to the present invention is then introduced into a prokaryotic cell, by carrying out any method known in the art, as for example by a temperature shock.

The following step is that of growing said prokaryotic cell in a suitable liquid culture medium, thus allowing the prokaryotic cell to produce the human pro-BDNF mutein according to the present invention.

In particular, the human pro-BDNF mutein according to the present invention is advantageously accumulated in its inactive, insoluble and non-folded form in the cytosol of prokaryotic cells, thus forming insoluble aggregates, known as "inclusion bodies".

The aggregation of said mutein in the inclusion bodies was completely unpredictable a priori, because it was unknown the effect of the replacement of the wild-type sequence set forth in SEQ ID NO:9 of the wild-type human pro-BDNF with the cleavage site of TEV, namely the sequence set forth in SEQ ID NO:10, on the production process of rhBDNF starting from said mutein, in particular in the crucial step of expression of said mutein in a prokaryotic expression system of said mutein.

Preferably, said inclusion bodies contain at from about 30% to about 50%, determined by RP-UPLC combined with Bradford assay, of human pro-BDNF mutein according to the invention, based on the total protein amount in the inclusion bodies.

To isolate said inclusion bodies, which comprises said human pro-BDNF mutein in the form of inactive, insoluble and non-folded protein, the prokaryotic cells are lysed (i.e. disrupted) following fermentation.

Said step of lysing the prokaryotic cells containing said inclusion bodies may be performed by conventional methods, e.g. by means of high-pressure homogenization, sonication or lysozyme (Rudolph, R., et al. (1997); Folding proteins. In: Creighton, T. E. (ed.): Protein Function: A Practical Approach. Oxford University Press, pp. 57-99).

Accordingly, in an embodiment of the present invention, said step i) comprises the steps of:
- providing a nucleic acid sequence encoding for a human pro-BDNF mutein according to the present invention;
- introducing said nucleic acid sequence into an expression vector, preferably a prokaryotic expression vector;
- introducing said expression vector into a prokaryotic cell;
- growing said prokaryotic cell in a suitable liquid culture medium, wherein said prokaryotic cell expresses the human pro-BDNF mutein according to the present invention, in form of inclusion bodies;
- lysing the prokaryotic cells and isolating said inclusion bodies.

After obtaining said inclusion bodies, which included inactive, insoluble and non-folded pro-BDNF mutein according to the present invention, the inclusion bodies are solubilized (i.e. dissolved) in a suitable denaturing solution, in order to disaggregate the inclusion bodies, and obtain free (i.e. not in an aggregated form) inactive, insoluble and non-folded human pro-BDNF mutein according to the present invention.

Accordingly, subsequently to the above-disclosed step i), a step ii) of solubilizing said inclusion bodies in a denaturing solution, thus obtaining a solution of denatured human pro-BDNF mutein, is carried out as disclosed below.

The denaturing solution preferably comprises a chaotropic agent, and/or a chelator, and/or a buffer, and/or a reducing agent.

Preferably, said chaotropic agent is a guanidinium salt, more preferably guanidinium hydrochloride.

Preferably, said buffer is a Tris buffer.

Preferably, said chelator is ethylenediaminetetraacetic acid (EDTA).

Preferably, said reducing agent is cysteine.

Moreover preferably, said denaturing solution comprises: 1M - 8 M, preferably 3M - 6 M guanidinium hydrochloride (Guanidinium-HCl); 0.05-0.2 M, preferably 0.1 M, Tris buffer; 2.5 mM - 10 mM, preferably 5 mM cysteine-HCl; and 5 mM - 15 mM, preferably 10mM, EDTA. After the solubilization of the inactive, insoluble and non-folded human pro-BDNF mutein, the mutein is refolded as disclosed below.

Accordingly, subsequently said step ii), a step iii) of diluting the solution of denatured human pro-BDNF mutein into a refolding solution is carried out as disclosed below, thus obtaining a correctly folded human pro-BDNF mutein.

For the refolding process, it is important to minimize the competing reactions of misfolding and aggregation.

Preferably, the inactive, insoluble and non-folded human pro-BDNF mutein is renatured in a refolding solution, which contains at least one chaperone, preferably Arginine, at least one metal chelator, preferably EDTA, and a redox shuffling system, preferably Cystine and/or Cysteine-HCl, said refolding solution having a pH comprised between 8.0 and 11.

More preferably, said refolding solution comprises: 0.5-1 M Arginine, preferably Arginine-HCl, 2-10 mM EDTA, 2-10 mM Cysteine HCl, 0.5-2 mM Cystine, pH 8.5-10.

Even more preferably, said refolding solution comprises: 0.75 M Arginine-HCl, 5 mM EDTA, 5 mM Cysteine HCl, 1 mM Cystine, pH 9.1.

In a preferred embodiment of the invention, said denaturing solution and/or the refolding solution contains no detergent.

In fact, the use of detergents is not necessary for the solubilization and/or folding of pro-BDNF mutein according to the present invention; this is advantageous, since certain detergents are comparatively aggressive chemical substances which pharmaceutical products should not comprise or should comprise in only small amounts, and therefore must be removed in an expensive manner. In other words, no detergents are used in the entire production method according to the invention, and the production method is therefore detergent-free.

After refolding, the thus-obtained solution may to be optionally clarified before loading onto a purification column; this can be done by any methods known in the art, for example, by filtration.

By the end of step iii), and the optional step of filtering, a correctly folded human pro-BDNF mutein is obtained in an aqueous solution, comprising said refolding solution.

Accordingly, the following step is step iv) of purifying the correctly folded human pro-BDNF mutein from the solution provided in step iii).

Such a step of purifying may be carried out by any of the known methods.

Preferably, the correctly folded pro-BDNF mutein obtained in step iii) is purified from the refolding solution via chromatography, more preferably by means of a mixed mode chromatography, most preferably wherein the chromatography column is a mixed mode material column with a synthetic affinity ligand, for example preferably 4-mercapto-ethyl-pyridine (MEP Hypercell; Pall). Advantages of this medium are that the binding is independent of the ionic strength, salt stacking is not necessary and higher flow rates to fasten the process are possible. Further, the elution is done by a pH-shift.

Other mixed mode material columns are known and could be used.

For example, but not limited to, MEP (Pall; affinity ligand is 4-Mercapto ethyl pyridine), HEA (Pall; affinity ligand: Hexylamino), PPA (Pall, affinity ligand: Phenylpropylamino), MBI (Pall; affinity ligand: 2-Mercapto-5benzamidazole sulfo acid), Capto MMC (GEHC), Capto adhere (GEHC; affinity ligand: N-benzyl-N-methyl ethanolamine), CHT hydroxyapatite (BioRad), CHT fluoroapatide (BioRad). The MEP, HEA, PPA, and MBI columns have a hydrophobic binding, where Capto MMC is a cation exchanger with mixed mode functionality and Capto adhere is an anion exchanger with mixed mode functionality. The BioRad columns are ion exchange columns with hydrophobic components.

Preferably, to avoid protein precipitation, the column may be eluted with an aqueous solution comprising urea, for example an aqueous solution comprising 1 M urea, thus advantageously facilitating the harvesting of the purified correctly folded pro-BDNF mutein. Accordingly, said step a) of the present process comprises the following steps:
i) expressing a human pro-BDNF mutein according to the present invention in inclusion bodies;
ii) solubilizing said inclusion bodies in said denaturing solution, thereby obtaining a solution of denatured human pro-BDNF mutein;
iii) diluting the solution of denatured human pro-BDNF mutein into said refolding solution, thus obtaining a correctly folded human pro-BDNF mutein,
iv) purifying the correctly folded human pro-BDNF mutein from the solution provided in step iii).

### - Step b) of the process according to the present invention

By the end of the above step a) of the process of the present invention, a purified correctly folded human pro-BDNF mutein is obtained, said pro-BDNF mutein being the precursor of the correctly folded, biologically active human BDNF according to the present invention. Thus, step b) of the present process is that of cleaving the pro-sequence of said human pro-BDNF mutein by a TEV protease as disclosed below, thus obtaining said correctly folded, biologically active rhBDNF.

As used herein, the acronym "TEV" is used to identify, depending upon the context, the wild-type Nuclear-inclusion-A endopeptidase (NIa) enzyme deriving from Tobacco Etch Virus ("wild-type TEV"), or a mutated version thereof ("mutated TEV protease").

Wild-type TEV, as well as the mutated version thereof, is a cysteine protease, and a member of the PA clan (Proteases of mixed nucleophile, superfamily A) of chymotrypsin-like proteases, with an apparent molecular weight of 28 kDa.

Preferably, said wild-type TEV is recombinantly expressed in a prokaryotic expression system, for example in *E*. *coli,* and more preferably it is further engineered to prevent autolysis (which typically occurs in TEV naturally-produced by Tobacco Etch Virus) and improve stability thereof.

Even more preferably, said wild-TEV also contains a 7x His-Tag for allowing efficient protein solubilization, and shows an optimal enzymatic activity between pH 6.0 and 9.0.

An example of said wild-type TEV is the wild-type TEV purchased from New England Biolabs, commercialized by Euroclone in Italy (ref BP8112S).

Preferably, said mutated TEV protease is recombinantly expressed in a prokaryotic expression system, for example in *E*. *coli,* and more preferably it is further engineered to prevent autolysis (which typically occurs in TEV naturally-produced by Tobacco Etch Virus) and improve stability thereof.

Even more preferably, said wild-TEV also contains a 7x His-Tag for allowing efficient protein solubilization, and shows an optimal enzymatic activity between pH 6.0 and 9.0.

Most preferably, as also in depth-discussed below, said mutated TEV protease has been engineered to recognize the wild-type TEV protease recognition site sequence, namely SEQ ID NO:10 (ENLYFQ), and to efficiently cleave after said sequence, regardless of the next amino acid (with the exception of proline) of the target protein, with high accuracy, thus leaving any undesired additional residues to the N-terminal of the target protein.

An example of said mutated TEV protease is the Numacut^{™} TEV protease purchased from Numaferm GmbH (ref P1065-100).

The cleavage site sequence cleaved by both wild-type TEV and a mutated TEV protease is herein identified as set forth in SEQ ID NO:10.

Even if the wild-type TEV generally recognizes the preferred amino acid sequence of SEQ ID NO: 10, and performs the proteolytic cleavage between Q at position P1, according to Table 1 (namely, the last one amino acid of the pro-sequence), and the following amino acid (namely, the first amino acid of the human BDNF), which is preferably a Ser (S, Serine) or Gly (G, Glycine) amino acid, the Experimental section of the present invention surprisingly demonstrated that said wild-type TEV can also efficiently cleaved between Q amino acid at P1 and a H (His, Histidine) amino acid as first amino acid of the BDNF sequence, as shown in SEQ ID NO:8.

Alternatively, a mutated TEV protease, according to the invention, may be used to carry out said step b) of the process of the present invention.

In particular, said mutated TEV protease recognizes the amino acid sequence of SEQ ID NO: 10, and performs the cleavage between Q at position P1, according to Table 1, and substantially any amino acid at position P1', according to Table 1, being indeed highly effective in the cleavage after Q at position P1, regardless of the amino acid at position P1', according to Table 1. In a preferred embodiment, said any amino acid at position P1' is any amino acid except Proline (Pro, P). An example of said mutated TEV protease according to the present invention may be Numacut^{™} TEV protease, provided by Numaferm GmbH. According to a preferred embodiment of the present invention, the preferred TEV protease is wild-type TEV.

Preferably, for performing the cleavage of human pro-BDNF mutein to rhBDNF in step b) of the present invention, the ratio of TEV protease to said pro-BDNF mutein is comprised between 1:2 and 1:500 *w*/*w* (weight/weight), more preferably between 1: 10 and 1: 250 *w*/*w,* most preferably is a ratio of 1:50 *w*/*w.*

Preferably, the cleavage occurs for a time range comprised between 20 hours and 100 hours, more preferably between 48 hours and 72 hours, at room temperature.

Under the conditions used in this invention, pro-BDNF mutein is substantially cleaved completely, and almost no pro-BDNF digestion by-products are formed. No aggregation was observed.

### - Step c) of the process according to the present invention

The rhBDNF obtained by the end of step b) is further purified, for example, by several chromatographic methods, in order to separate the rhBDNF from the TEV protease and the released pro-peptide, obtained by the end of the digestion step b).

Any methods known in the art for protein purification can be used.

Most preferred purification methods are chromatographic purifications, for example by Sepharose columns (e.g. SP Sepharose HP, Phenyl Sepharose low substitution FF, Agarose-based ion exchange chromatography resins, such as for example Praesto^{™} SP65 (Purolite)).

The final product, namely a correctly-folded, biologically active rhBDNF obtained by the process according to the present invention, starting from the human pro-BDNF mutein according to the present invention, is preferably characterized by a purity of at least 94.5 %, measured by RP-UPLC.

Said final product rhBDNF may also comprise at least one of the following:
- oxidized rhBDNF forms in a total concentration not higher than 4 % (w/w), preferably of about 3.5 % w/w:
- acetylated rhBDNF forms in a total concentration not higher than 2.5 % (w/w), preferably of about 2 % w/w.

Another aspect of the present invention is the use of a pro-BDNF mutein according to the present invention, for the preparation of a rhBDNF, preferably a correctly folded, biologically active rhBDNF as disclosed above.

The following examples are put forth so as to provide those of ordinary skill in the art with a complete disclosure and description of how to make and use the methods and compositions of the invention, and are not intended to limit the scope of what the inventors regard as their invention.

### EXAMPLES

### Example 1

### - Cloning of the DNA encoding a human pro-BDNF mutein according to the present invention into an expression vector and insertion into E. coli

A synthetic gene (corresponding to a human pro-BDNF mutein according to the present invention) consisting of SEQ ID NO: 11 was prepared, with triplet codons optimized for their expression in *Escherichia coli,* and it was inserted into a pET28 expression vector (Novagen).

The thus-obtained expression vector was inserted into *Escherichia coli* BL21 (DE3) strain by temperature shock according to supplier instructions (BL21 (DE3) Chemi Competent cells ref c2527H; New England Biolabs).

### - Fermentation and induction of expression of the mutein.

A single colony of the recombinant *E. coli* strain was inoculated in 30 ml of Terrific Broth (TB) medium (Sigma Aldrich) containing 50 µg/ml of kanamycin, which was then incubated under agitation at 28°C (batch phase) for 16 hours.

The day after, 600 ml of fresh TB, containing 50 µg/ml kanamycin, were inoculated with 20 ml of the overnight culture, and culture was run at 30°C until OD₆₀₀ₙₘ reached 32, fed-batch phase was started, and temperature increased to 37°C; after one hour 1mM Isopropyl β- d-1-thiogalactopyranoside (IPTG) was added to the medium to induce the expression of the recombinant protein and the culture was continued on for three further hours for a 900 mL final volume.

The fermentation has been carried out at pH 7.0.

To monitor the over-expression of the target protein during the fermentation process, samples were analyzed by means of SDS-PAGE before and after induction. As human pro-BDNF mutein possesses three intrachain disulfide bridges, the protein was accumulated in the cytoplasm of the cells in form of inclusion bodies (iBs).

### - Isolation and solubilization of inclusion bodies

The cells were harvested by centrifugation, resuspended at 1.5 weight/volume in Lysis buffer (0.1 M tris, 0.025 M EDTA, pH 7.0) and homogenized with a NS1001L2K Niro Soavi high-pressure homogenizer for the equivalent of four cycles at 800 bars. The homogenate obtained was diluted 1:0.5 with a preparation of Brij 35 (60gr/L) and stirred for 30 minutes. The pellets, which comprised the inclusion bodies, were isolated by centrifugation (8500 rpm, Sorval GS3 rotor), washed twice with Tris 0.1 M pH 7.0 at 0.4 to 1 weight/volume. The mixture was centrifuged (8500 rpm, Sorval GS3 rotor) and the pellets were dissolved for 3 hours, at room temperature, in a denaturing solution consisting of: Guanidinium-HCl 4 M, Tris 0.1 M, Cysteine-HCl 5 mM, EDTA 10 mM , after dissolution pH has been adjusted to 4.

### - Refolding of the pro-BDNF mutein

To prepare the correctly folded human pro-BDNF mutein according to the invention, 360 mL of the solubilized material obtained in the previous step was refolded at 4°C into at least 5 L of the following refolding solution (Table 2):

**Table 2: Refolding solution composition**

| **Component** | **Concentration** | **MW (gr/mole)** | **gr/L** |
|---|---|---|---|
| 0.75 M Arg-HCl | 0.75 M | 210,66 | 158 |
| 5 mM EDTA | 5 mM | 372,24 | 1.86 |
| 5 mM Cysteine HCl | 5 mM | 175,74 | 0.88 |
| 1 mM Cystine | 1mM | 240,3 | 0.24 |
| pH 9.1 | | | |

At the end of said refolding step, the human pro-BDNF mutein assumes a correctly folded conformation.

The concentration of guanidine after dilution into the refolding solution was below 0.3 M. The performance of the refolding reaction was analyzed by RP-UPLC and was estimated to be 52 % of the initially solubilized protein.

### - Purification of the mutein from the refolding solution

In order to purify the correctly folded human pro-BDNF mutein from the refolding solution, a hydrophobic induced charge chromatography was carried out using a column with the synthetic affinity ligand 4-mercapto-ethyl-pyridine (MEP).

Before loading the solution comprising said correctly refolded human pro-BDNF mutein, obtained by the previous step the column was equilibrated with 5 column volumes of 0.75 M Arginine HCl, pH 9.1.

Then, said solution comprising the correctly refolded human pro-BDNF mutein was loaded onto the previously equilibrated MEP column. The column is then first washed with 3 column volumes of equilibration buffer, then with Tris 10 mM pH 8.0 for further 3 column volumes. Subsequently, said mutein was eluted to baseline by using an aqueous solution consisting of: 1 M urea, 50 mM Citric acid, at pH 4.

The thus-obtained harvested solution comprising said mutein has then been concentrated and buffer exchanged with a solution consisting of Tris 50 mM, 0.6 M arginine and 0.5 mM EDTA, pH 7 which is a buffer compatible with TEV protease digestion, by carrying out a tangential flow filtration (TFF) on a 10 kDa cassette Omega Centramate from Pall.

At the end of the above TFF, a solution (Tris 50 mM, 0.6 M arginine and 0.5 mM EDTA at pH 7) comprising said correctly refolded human pro-BDNF mutein at a concentration of 4 mg/mL was obtained.

### - Cleavage of the human pro-BDNF mutein by wild-type TEV protease to obtain mature BDNF

The solution obtained in the previous step, comprising the correctly refolded human pro-BDNF mutein at a concentration of 4 mg/mL, was incubated with wild-type (wt) TEV protease (Euroclone), to perform the digestion of the mutein, thus cleaving the pro-sequence from the pro-BDNF mutein according to the present invention.

In particular, half of said solution was incubated for 24 hours at room temperature (21°C ± 2°C), wherein the ratio of wt TEV protease to said pro-BDNF mutein was of 1 to 50.

The other half of said solution was incubated for 48 hours at room temperature (21°C ± 2°C), wherein the ratio of wt TEV protease to said pro-BDNF mutein was of 1 to 50.

The above enzymatic digestions were stopped by adding an aqueous solution comprising 2 M urea.

At the end of said digestion periods, the digested products have been analyzed by RP-UPLC and by SDS-PAGE.

The Reverse-Phase UPLC (RP-UPLC) analysis was performed as described below.

UPLC analysis was performed on a Waters Acquity UPLC H class liquid chromatograph equipped with a PDA eλ detector (Waters). The liquid chromatography separation of the protein samples and impurities was performed using a C4 column (2.1 x 100 mm, particle size of 1.7 µm) maintained at 60°C. Mobile phase A was a mixture of 0.1% (v/v) trifluoracetic acid and MilliQ water, and mobile phase B was a mixture of 0.1% (v/v) trifluoracetic acid and Acetonitrile, in a flow gradient of 0.3 mL/min. The injection volume was 2-5 µL and ultraviolet detection was carried out at 220 nm. Each sample to be tested was diluted if needed, in formulation buffer and the assay was determined against an in-house rhBDNF working standard protein using response factor or a calibration curve.

The RP-UPLC gradient is reported below:

| **Time (min)** | **Flow (mL/min)** | **% A** | **% B** |
|---|---|---|---|
| 0.00 | 0.300 | 80.0 | 20.0 |
| 1.00 | 0.300 | 80.0 | 20.0 |
| 7.00 | 0.300 | 60.0 | 40.0 |
| 7.10 | 0.300 | 30.0 | 70.0 |
| 9.00 | 0.300 | 30.0 | 70.0 |
| 9.10 | 0.300 | 80.0 | 20.0 |
| 12.00 | 0.300 | 80.0 | 20.0 |

The SDS-polyacrylamide (SDS-PAGE) gel analysis was performed as described below.

The SDS-PAGE gel was prepared according to the following protocol.

The SDS-PAGE analysis was conducted under reducing conditions. Samples were mixed with NuPAGE 4x LDS Sample Buffer (Invitrogen) and NuPAGE 10x Reducing Agent (Invitrogen), and successively denatured at 100°C for 10 minutes before loading in the gel. About 2.33 µg of rhBDNF Reference Standard (Dompé in-house) were loaded on the gels, while for the analytical test samples (fractions 1-10) the loaded amount depended on their respective measured concentration. NuPAGE Novex 4-12%, Bis-Tris Polyacrylamide gels (Invitrogen) were assembled in XCell Surelock Mini Cell Apparatus (Invitrogen) and the internal chamber was filled with 200 mL of 1x Running Buffer with the addition of antioxidants (NuPAGE Antioxidant (Invitrogen)), while the outer chamber was filled with about 300 mL of 1x Running Buffer.

The 1x Running Buffer was prepared from NuPAGE 20x MES SDS Running Buffer stock solution (Invitrogen). Gels were run at a constant voltage of 200 V for 35 minutes; afterwards, gels were fixed and stained with NOVEX Colloidal Blue Staining Kit (Invitrogen).

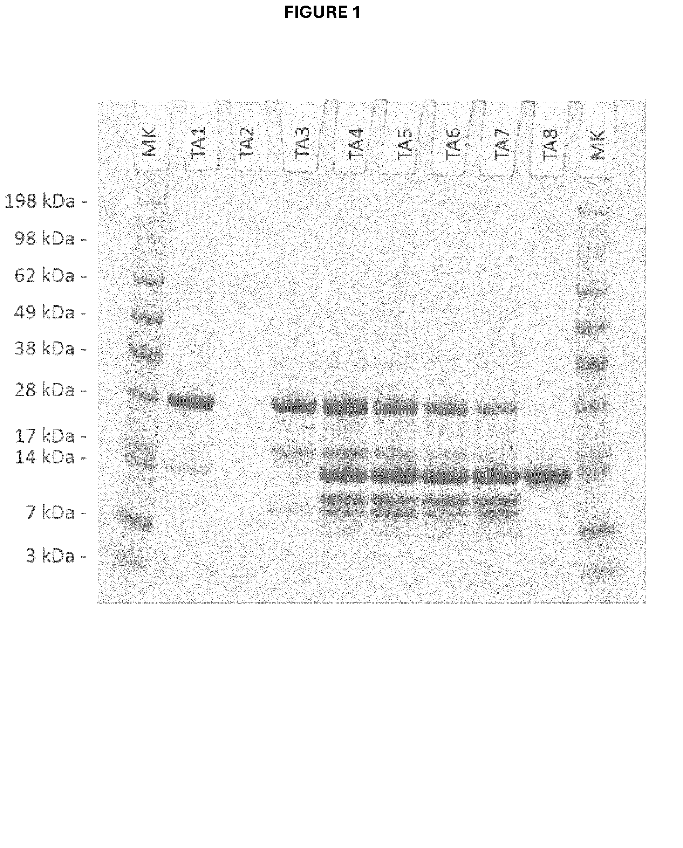

Figure 1 reports the results of the electrophoresis analysis (also comprising the results of the electrophoresis analysis of Example 2, for experimental convenience), according to the gel lanes of Table 3:

**Table 3: Gel lanes and samples of Figure 1.**

| **GEL LANE** | **SAMPLE** | |
|---|---|---|
| MK | Protein Molecular Weight Markers, | |
| | SeeBlue Plus2 Prestained Standard (Invitrogen) | |
| TA1 | Recombinant Human pro-BDNF | |
| | (Reference Standard) | |
| TA2 | Blank | |
| TA3 | T0 | |
| | (sample before enzymatic digestion) | |
| TA4 | Numacut^{™} TEV protease (Numaferm GmbH) | See Example 2. |
| | Enzymatic Digestion Time: 24 hours | |
| TA5 | Numacut^{™} TEV protease (Numaferm GmbH) | See Example 2. |
| | Enzymatic Digestion Time: 48 hours | |
| TA6 | Wild-type TEV protease (Euroclone spa) | |
| | Enzymatic Digestion Time: 24 hours | |
| TA7 | Wild-type TEV protease (Euroclone spa) | |
| | Enzymatic Digestion Time: 48 hours | |
| TA8 | rhBDNF Reference Standard | |
| MK | Protein Molecular Weight Markers, | |
| | SeeBlue Plus2 Prestained Standard (Invitrogen) | |

Said "rhBDNF" reference standard and "human pro-BDNF" reference standard were obtained in-house, and are highly purified products, having indeed a purity higher than 90% and 94%, respectively.

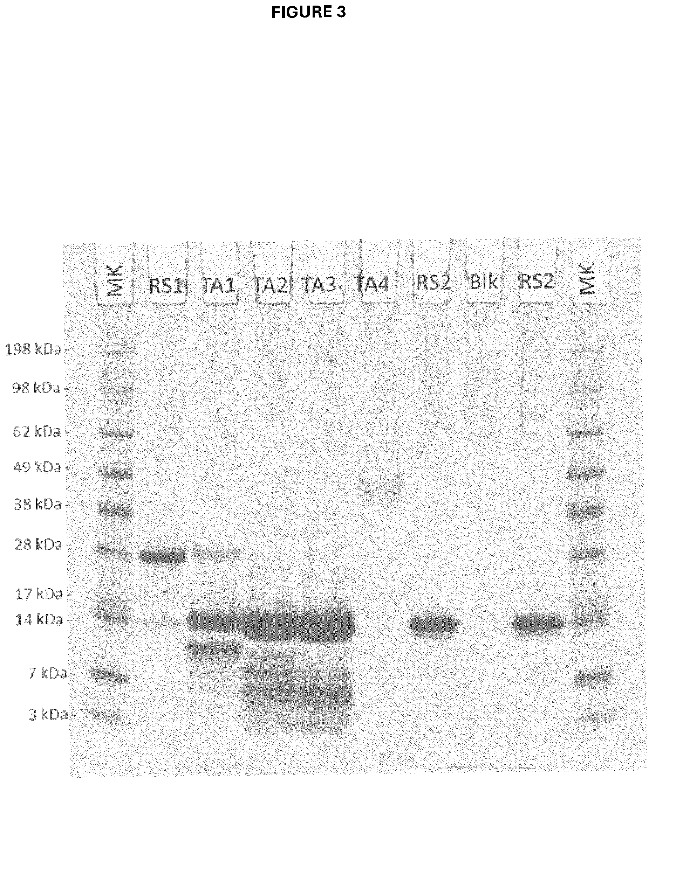

These in-house produced "rhBDNF" and Human pro-BDNF reference standards were also used in Figures 3, 4 and 5.

As shown in Figure 1, the enzymatic digestion of the human pro-BDNF mutein according to the present invention is digested by the above-mentioned wild-type TEV protease, both at 24 hours and 48 hours of incubation time.

In fact, in TA6 and TA7 of Figure 1, it is shown a thick and well-visible band with a molecular weight (MW) of 13.5 kDa, which corresponds to the mature rhBDNF, and very less visible bands with a MW of 26 kDa, which correspond to the (entire, not digested) pro-BDNF mutein. The bands with a MW of 16 kDa and 9 kDa shown in Figure 1 correspond to two E. *coli* contaminant proteins, respectively.

Surprisingly, no hypo-digested and hyper-digested rhBDNF forms were detectable, thus demonstrating that the digestion by wild-type TEV protease was highly specific, and no by-products from the digestion of the pro-BDNF mutein according to the present invention were obtained.

This result means that the above-disclosed enzymatic digestion of the mutein according to the present invention, by wild-type TEV protease, results in an advantageous and unexpected high yield of mature rhBDNF.

As TA7 (digestion performed for 48 hours) of Figure 1 shows a thicker band corresponding to rhBDNF compared to TA6 (digestion performed for 24 hours), the following steps of the process according to the present invention were carried out starting from the above-disclosed solution comprising rhBDNF obtained by wild-type TEV digestion performed for 48 hours.

### - Purification step of rhBDNF

After TEV digestion, the pH of said solution comprising rhBDNF was adjusted to a value of 5.0 by addition of an acidic (HCl) solution, and the thus-obtained solution was first diluted 1 to 2 with citric acid 50mM, 2 M Urea pH 5 (to decrease conductivity), and then loaded onto a chromatographic column packed with the Cytiva SP-HP resin to deplete TEV protease, undigested pro-BDNF, the released pro-peptide, and further impurities.

The column was previously equilibrated with 50 mM Citric acid, 125 mM arginine, 0.5 mM EDTA and 1 M urea pH 5.0.

After loading rhBDNF comprising solution, the thus-equilibrated column was then washed with the previously described buffer, and the rhBDNF was then eluted in 25 mM Phosphate buffer, pH 6.5 increasing the salt concentration with a step gradient.

rhBDNF was eluted in two well separated peaks, the first one corresponding to dimers and the second one to multimers as demonstrated by Dynamic Light Scattering measurements (data not shown), which revealed that, in agreement with tetramer conformation, the second peak particles diameter is exactly the double of those in the first one.

As TEV protease and rhBDNF isoelectric points are sufficiently different, the TEV protease did not co-elute with rhBDNF, and was therefore easily separated from rhBDNF and pro-BDNF digestion by-products.

Material corresponding to the first peak (dimers) has then been adjusted to 1.9 M NaCl and loaded on a chromatographic column packed with Phenyl Sepharose low substitution resin (provided by Cytiva).

The column was equilibrated with 25 mM Phosphate buffer, 2M NaCl pH 6.5.

After loading and washing, with the equilibration buffer (25 mM Phosphate buffer, 2M NaCl pH 6.5), the column has been eluted by dropping buffer salt concentration to 0.4 M NaCl, and the fraction comprising rhBDNF was eluted as a single peak.

The RP-UPLC analysis carried out on this fraction confirmed that the rhBDNF was pure at about 94.5 % w/w; this fraction also contained product-related impurities, mainly oxidized rhBDNF forms in a total concentration of 3.5 % w/w, and acetylated rhBDNF forms in a total concentration of 2 % w/w.

### - Test for the biological activity of rhBDNF

The biological activity of the rhBDNF, obtained according to the above, was tested and compared to the biological activity of a standard rhBDNF, by using rat C6 glioma cells expressing high levels of BDNF TrkB receptor (Xiong J. et al., Oncology Letters 2015; 10:223-227).

In this experiment, the rhBDNF obtained as above was named "rhBDNF-TEV", and the standard rhBDNF was named "rhBDNF".

It was demonstrated that through this receptor, mature BDNF promotes C6 cell growth and survival in a dose-dependent manner (Xiong J. et al., Oncology Reports 2013; 30(6):2719-2724).

Therefore, C6 cells were plated in 96-well microplates at a density of 5000 cells/well and treated with rhBDNF-TEV or rhBDNF at a concentration ranging from 0.23 to 9 µg/mL, to test rhBDNF-induced proliferation effect (Cell proliferation Assay, MTS assay).

After 48h incubation, the CellTiter 96^{®} AQueous One Solution reagent (Promega, Madison, WI, USA, code # G3581) was added to the microwell plates to measure and determine the biological activity of rhBDNF-TEV and rhBDNF from the number of viable C6 cells.

The EC₅₀, corresponding to the concentration of rhBDNF required to induce 50% proliferation of the cells, was determined for rhBDNF-TEV and rhBDNF produced according to the above by using the GraphPad software.

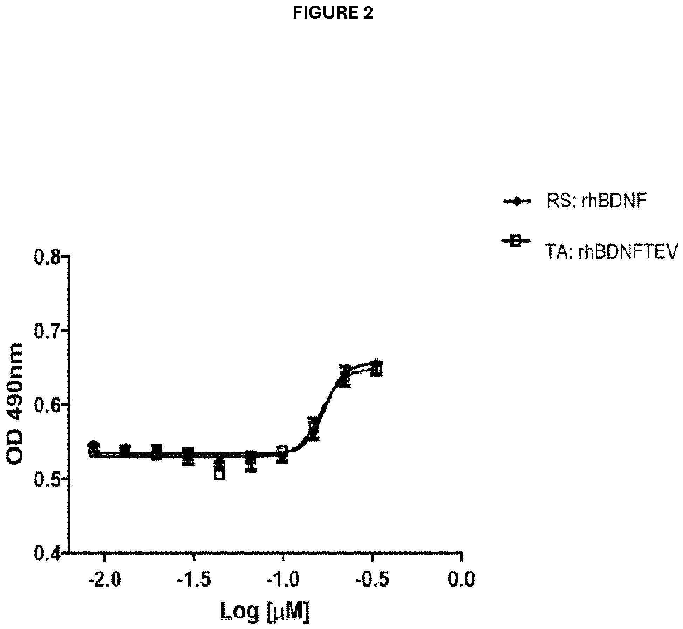

The results are shown in Figure 2 and demonstrate the substantial identity between the proliferation curve related to the standard rhBDNF and the proliferation curve related to rhBDNF-TEV.

These results mean that the biological activity of the rhBDNF obtained as above is completely comparable to that of the standard rhBDNF.

Regarding the specific EC₅₀ values, the EC₅₀ of the (standard) rhBDNF was 0.1716 µM, and the EC₅₀ of the rhBDNF-TEV was 0.1624 µM.

Thus, besides the above-discussed proliferation curves, the EC₅₀ values of rhBDNF-TEV and rhBDNF are substantially the same, thus confirming the rhBDNF-TEV is biologically active as the standard rhBDNF.

### Example 2

Another example of the process according to the present invention is disclosed below. The steps of cloning, fermentation, isolation and solubilization of inclusion bodies, refolding of the pro-BDNF mutein, and purification of the pro-BDNF mutein were carried out according to Example 1.

The cleavage step was carried out as follows.

The solution comprising pro-BDNF at a concentration of 4 gr L⁻¹ was incubated with Numacut^{™} TEV protease (Numaferm GmbH), to perform the digestion of the mutein, thus cleaving the pro-sequence from the pro-BDNF mutein according to the present invention. In particular, half of said solution was incubated for 24 hours at room temperature (21°C ± 2°C), wherein the ratio of Numacut^{™} TEV protease to said pro-BDNF mutein was of 1 to 50.

The other half of said solution was incubated for 48 hours at room temperature (21°C ± 2°C), wherein the ratio of Numacut^{™} TEV protease to said pro-BDNF mutein was of 1 to 50. The above enzymatic digestions were stopped by adding an aqueous solution comprising 2 M urea.

At the end of said digestion periods, the digested products have been analyzed by RP-UPLC and by SDS-PAGE, according to Example 1.

Figure 1 reports the results of the electrophoresis analysis according to the TA4 and TA5 gel lanes of the above-reported Table 3.

As shown in Figure 1, the enzymatic digestion of the human pro-BDNF mutein according to the present invention is successfully completed by the above-mentioned Numacut^{™} TEV protease, both at 24 hours and 48 hours of incubation time.

The results of RP-UPLC analysis of enzymatic digestion of human pro-BDNF mutein according to the present invention carried out with Wild-type TEV protease (Example 1) and Numacut^{™} TEV protease are reported in the Table 4 below:

**Table 4: RP-UPLC analysis of TEV WT and TEV Numacut enzymatic digestions.**

| Digestion time (h) | Enzyme | BDNF | |
|---|---|---|---|
| | | µg/ml | Area % |
| 0 | N/A | 0 | 0 |
| 24 | Numacut | 1349,4 | 33,8 |
| 24 | WT TEV | 1852,4 | 49,9 |
| 48 | Numacut | 1617,3 | 43,8 |
| 48 | WT TEV | 2109 | 58,4 |

Surprisingly, these results were highly similar the those obtained by carrying out the digestion by wild-type TEV protease, as the bands obtained in said TA4 and TA5 substantially correspond to the bands in TA6 and TA7, as reported in Example 1.

Therefore, these results demonstrate that Numacut^{™} TEV and wild-type TEV proteases are equally effective in cleaving, with high specificity, the pro- sequence from the human pro-BDNF mutein according to the present invention, thus obtaining a high yield of mature rhBDNF, with no detectable pro-BDNF digestion by-products (i.e. hypo- and hyper-digested rhBDNF forms).

### Example 3

As the process according to the present invention was unexpectedly efficient in obtaining a correctly folded, biologically active rhBDNF starting from the above human pro-BDNF mutein comprising a single recognition site sequence of a TEV protease, according to the above Example 1 and Example 2, the inventors tested other proteases recognizing a single cleavage site for obtaining a correctly folded, biologically active rhBDNF, as disclosed below.

### - Enterokinase

The enterokinase recognition site is the amino acid sequence of **SEQ ID NO: 12** (DDDDK), and the cleavage occurs between the K amino acid and any following amino acid.

Thus, a human pro-BDNF mutein comprising said recognition site at amino acid position P5 - P1, according to Table 1 (i.e. a D at P5, a D at P4, a D at P3, a D at P2, and a K at P1 position) was obtained as follows.

A synthetic gene (corresponding to a human pro-BDNF mutein comprising said recognition site) was prepared with triplet codons optimized for their expression in *Escherichia coli,* and inserted into an expression vector, which was subsequently transformed into an *Escherichia coli* strain, as reported in Example 1.

The steps of fermentation, isolation and solubilization of inclusion bodies, and refolding of the human pro-BDNF mutein were carried out as reported in Example 1.

The purification of the thus-obtained pro-BDNF mutein were carried out on a MEP column, according to Example 1, and then a TFF was performed to concentrate the pro-BDNF mutein to 4 mg/mL and buffer exchange it with the recommended buffer (20 mM Tris-HCl, 50 mM NaCl, 2 mM CaCl₂ pH 8.0, to which 1 M urea has been added to avoid pro-BDNF precipitation) for carrying out the enterokinase digestion in the subsequent step.

However, during said concentration by TFF, the solution comprising said pro-BDNF mutein unexpectedly became very cloudy at a concentration of about 2.27 mg/mL (determined by measuring the solution absorbance at 280nm), and it was impossible to reach the desired pre-established concentration of 4 mg/ml.

As enzymatic digestion typically depends on the substrate concentration, such a diluted concentration could have a negative effect on the digestion kinetic.

The solution comprising pro-BDNF at 2.27 mg/mL has been digested with enterokinase protease (provided by Euroclone, ref BP8070L) at 22°C, according to Table 5:

**Table 5: RP-UPLC analysis of Enterokinase digestion.**

| **Digestion time** | **BDNF µg/mL** | **% BDNF area** | **% Pro-BDNF area** |
|---|---|---|---|
| 24 hours | 817,1 | 40,6 | 0 |
| 48 hours | 497,4 | 23,3 | 0 |

At 48 hours, rhBDNF concentration unexpectedly decreases; without being bound to a theory or principle, the inventors hypothesized that such a decrease may be due to rhBDNF precipitation in this specific digestion buffer, despite the addition of 1 M Urea (indeed, pro-peptide digestion by the enterokinase protease typically generates small hydrophobic peptides, which tend to aggregate in this buffer, and finally precipitate after 48 hours, pulling down the correctly processed mature BDNF).

A further explanation may be a non-specific (and highly undesired) enterokinase digestion of mature rhBDNF.

Digested samples have been analyzed by SDS-PAGE (according to Example 1) for further characterization, and the results are shown in Figure 3, according to the gel lanes of the below Table 6:

**Table 6: Gel lanes and samples of Figure 3.**

| **GEL LANE** | **SAMPLE** |
|---|---|
| MK | Protein Molecular Weight Markers, |
| | SeeBlue Plus2 Prestained Standard (Invitrogen) |
| RS1 | Recombinant human pro-BDNF |
| | Reference Standard |
| TA1 | Enterokinase protease |
| | Enzymatic Digestion Time: 5 hours |
| TA2 | Enterokinase protease |
| | Enzymatic Digestion Time: 24 hours |
| TA3 | Enterokinase protease |
| | Enzymatic Digestion Time: 48 hours |
| TA4 | Enterokinase protease |
| | (10µL of stock solution) |
| RS2 | rhBDNF Reference Standard |
| Blk | Blank |
| RS2 | rhBDNF Reference Standard |
| MK | Protein Molecular Weight Markers, |
| | SeeBlue Plus2 Prestained Standard (Invitrogen) |

As shown in Figure 3, at 24 hours, the pro-sequence of pro-BDNF mutein was already completely digested, and gel lanes TA2 (24-hours digestion) and TA3 (48-digestion) clearly show that the mature rhBDNF was heavily contaminated by pro-BDNF digestion by-products, which are characterized by a molecular weight lower than that of rhBDNF.

This means that the protease not only has cleaved the pro-sequence from pro-BDNF mutein at its recognition site (as expected),but also has carried out undesired not-specific digestion(s) of the pro-sequence itself, thus producing a collection of undesired peptide fragments, in particular during the digestion performed for 48 hours.

Without being bound to a theory or principle, the inventors hypothesized that said undesired not-specific digestion(s) may be due to the similarity of several regions of the pro-sequence of pro-BDNF to the specific cleavage site of SEQ ID NO: 12.

Such as regions of the pro-sequence of pro-BDNF similar to the specific cleavage site recognized by enterokinase protease (represented by the SEQ ID NO:12) may be the ones reported in the following Table 7:

**Table 7: Examples of regions in the pro-sequence of human pro-BDNF similar to the specific cleavage site recognized by enterokinase protease (the amino acids similar to the canonical enterokinase cleavage site have been reported in bold and underlined).**

| **P5** | **P4** | **P3** | **P2** | **P1** | **Position in the pro-sequence of human pro-BDNF** | |
|---|---|---|---|---|---|---|
| V | N | G | P | **K** | 32 | **SEQ ID NO: 13** |
| **D** | **E** | **D** | Q | **K** | 58 | **SEQ ID NO: 14** |
| **E** | **E** | N | N | **K** | 67 | **SEQ ID NO: 15** |
| L | **E** | **E** | Y | **K** | 96 | **SEQ ID NO: 16** |
| **D** | **D** | **D** | **D** | **K** | 130 (Enterokinase cleavage site) | **SEQ ID NO: 12** |

Thus, the process according to the present invention for the production of a rhBDNF, starting from a human pro-BDNF mutein comprising a cleavage site recognized by enterokinase protease, as disclosed above, has several drawbacks.

First of all, the desired target concentration of said pro-BDNF mutein cannot be reached, thus negatively affecting the digestion step; moreover, enterokinase unexpectedly cleaved the pro-sequence, thus performing non-specific and undesired cleavages.

Consequently, the thus-obtained mature rhBDNF is heavily contaminated by pro-BDNF digestion by-products, and it is therefore poor in quality (i.e. purity) and yield.

### - Factor Xa

The Factor Xa recognition site is the amino acid sequence of **SEQ ID NO: 13** (IEGR), and the cleavage occurs between the R amino acid and any following amino acid.

Thus, a human pro-BDNF mutein comprising said recognition site at amino acid position P4 - P1, according to Table 1 (i.e. a I at P4, a E at P3, a G at P2, and a R at P1 position) was obtained as follows.

A synthetic gene (corresponding to a human pro-BDNF mutein comprising said recognition site) was prepared with triplet codons optimized for their expression in *Escherichia coli,* and it was inserted into an expression vector, which was subsequently transformed into an *Escherichia coli* strain, according to Example 1.

The steps of fermentation, isolation and solubilization of inclusion bodies were carried out according to Example 1.

The refolding step of the pro-BDNF mutein was carried out at 4°C in the refolding solution of Table 2, according to Example 1.

Then, the thus-obtained solution was filtrated and purified on a MEP column, according to Example 1.

The purified solution was then buffer exchanged with 20 mM Tris-HCl (pH 8.0, 25°C) 100 mM NaCl, 2 mM CaCl₂ and 1% Triton X 100 (as Factor Xa is sensitive to urea and arginine, 1% triton X 100 was added to the enzymatic buffer to prevent protein precipitation), and concentrated, by a TFF, thus obtaining a solution comprising pro-BDNF at 2.8 mg/mL. Said solution was then digested for 24 and 48 hours at 22°C by Factor Xa (New England Biolabs ref BP8010L), at both ratio of Factor Xa to pro-BDNF mutein of 1:50 and 1:25. The digestion results have been analysed by RP-UPLC and SDS-PAGE, as disclosed above (Example 1).

The results of said RP-UPLC analysis are reported below in Table 8:

**Table 8: RP-UPLC results of the digested products by Factor Xa.**

| **F.xa/Pro-BDNF Ratio** | **24 hours digestion** | | **48 hours digestion** | |
|---|---|---|---|---|
| | BDNF µg/mL | % BDNF Area | BDNF µg/mL | % BDNF Area |
| 1/25 | 81,9 | 4,7 | 84,4 | 4,8 |
| 1/50 | 56,9 | 3,1 | 53,8 | 2,9 |

The SDS-PAGE was carried out according to Example 1.

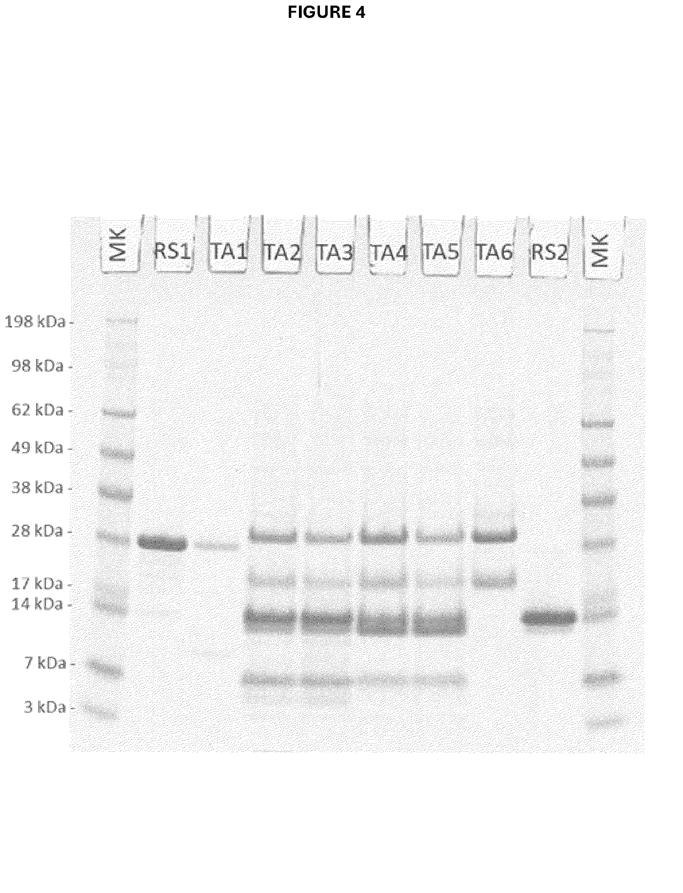

The results of the SDS-PAGE analysis are reported in Figure 4, in which the gel lanes are according to the following Table 9:

**Table 9: Gel lanes of Figure 4.**

| **GEL LANE** | **SAMPLE** |
|---|---|
| MK | Protein Molecular Weight Markers, |
| | SeeBlue Plus2 Prestained Standard (Invitrogen) |
| RS1 | Human pro-BDNF |
| | Reference Standard |
| TA1 | T0 |
| | (sample before enzymatic digestion) |
| TA2 | Factor Xa - ratio 1/25 |
| | Enzymatic Digestion Time: 24 hours |
| TA3 | Factor Xa - ratio 1/50 |
| | Enzymatic Digestion Time: 24 hours |
| TA4 | Factor Xa - ratio 1/25 |
| | Enzymatic Digestion Time: 48 hours |
| TA5 | Factor Xa - ratio 1/50 |
| | Enzymatic Digestion Time: 48 hours |
| TA6 | Factor Xa |
| RS2 | rhBDNF Reference Standard |
| MK | Protein Molecular Weight Markers, |
| | SeeBlue Plus2 Prestained Standard (Invitrogen) |

As shown in Figure 4, as well as Table 8, already at 24 hours the pro-BDNF mutein is completely digested even with the lower protease concentration, but surprisingly the mutein is not processed as expected.

In fact, if some correctly processed BDNF can be evidenced, on the SDS-PAGE or by RP-UPLC analysis, an important part of the mutein is converted into a hyperdigested form having a molecular weight very close to the mature rhBDNF.

Therefore, Factor Xa has unexpectedly cleaved the pro-BDNF mutein in an unspecific manner, thus producing a mature rhBDNF heavily contaminated by an undesired hyperdigested form.

### - Furin

Furin is the natural protease of human wild-type pro-BDNF, and it cleaves the pro-sequence of the protein, thus obtaining the mature human wild-type BDNF.

Therefore, it is expected that this enzyme is able to effectively cleave the pro-sequence of human pro-BDNF, without any hyper- or hypo digested BDNF variant.

A synthetic gene corresponding to the human wild-type pro-BDNF was prepared with triplet codons optimized for their expression in *Escherichia coli,* and inserted into an expression vector, which was subsequently transformed into an *Escherichia coli* strain, according to Example 1.

The steps of fermentation, isolation and solubilization of inclusion bodies, refolding, filtration and purification on a MEP column were performed according to Example 1.

To buffer exchange with a suitable digestion solution and concentrate the thus-obtained solution comprising wild-type pro-BDNF, a TFF (10 kDa cassette) was carried out, in order to obtain a wild-type pro-BDNF concentration of 4 mg/mL.

In particular, said digestion solution consisted of: 20 mM HEPES, 0.1% Triton X-100, 1 mM CaCl₂, 0.03 mM DTT and 1M urea (to prevent aggregation), pH 7.0.

However, even in the presence of 1M urea and 0.1% Triton x-100, the wild-type pro-BDNF aggregated during the concentration step, and the final concentration of wild-type pro-BDNF obtained was only of 0.42 mg/mL.

As the thus-obtained solution was also very cloudy, a filtration step was carried out on a Stericup (0.22 µm), and the final concentration of wild-type pro-BDNF in the solution to be digested was estimated at 0.3 mg/mL.

5 mL of the solution comprising wild-type pro-BDNF at 0.3 mg/mL was digested with furin protease (62.5µl corresponding to 125 units) for 24 and 48 hours at 22°C.

The digestion results were analysed by RP-UPLC and SDS-PAGE, carried out according to Example 1, as reported below.

The results of the RP-UPLC analysis are reported in the below Table 10:

**Table 10: RP-UPLC results of digested products by furin.**

| **Digestion time** | **BDNF µg/mL** | **% BDNF area** |
|---|---|---|
| 24 hours | 184 | 14,3 |
| 48 hours | 208 | 13,7 |

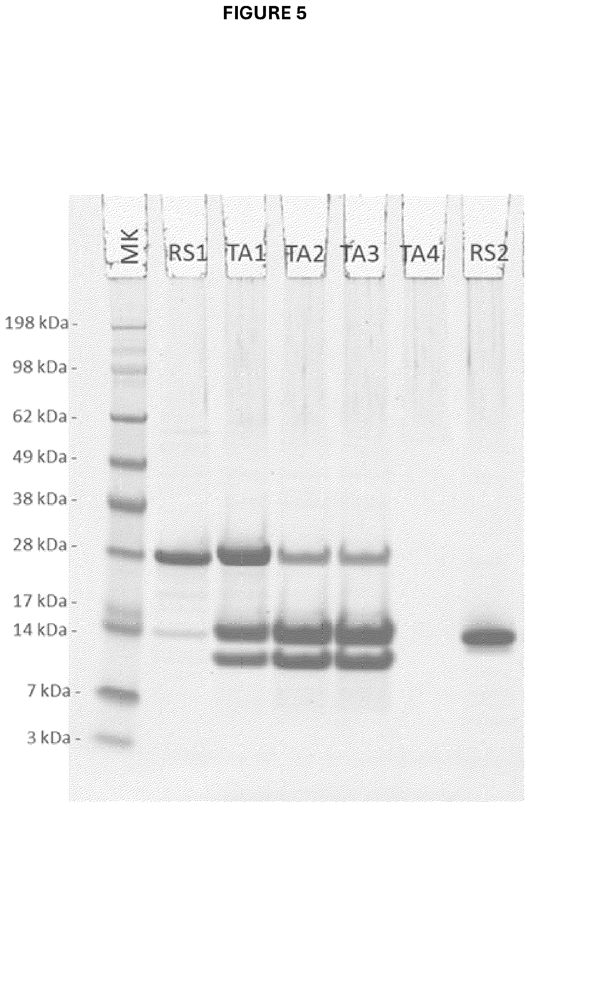

The SDS-PAGE results were reported in Figure 5, according to the gel lanes of Table 11:

**Table 11: Gel lanes of Figure 5.**

| **GEL LANE** | **SAMPLE** |
|---|---|
| MK | Protein Molecular Weight Marker, |
| | SeeBlue Plus2 Prestained Standard (Invitrogen) |
| RS1 | Recombinant Human pro-BDNF |
| | Reference Standard |
| TA1 | Furin |
| | Enzymatic Digestion Time: 5 hours |
| TA2 | Furin |
| | Enzymatic Digestion Time: 24 hours |
| TA3 | Furin |
| | Enzymatic Digestion Time: 48 hours |
| TA4 | Furin |
| RS2 | rhBDNF Reference Standard |

Even if the digestion of wild-type pro-BDNF by furin resulted, as expected, in the formation of mature BDNF, Figure 5 clearly shows that the digestion of wild-type pro-BDNF was not completed by furin even after 48 hours of digestion.

Therefore, the digestion by furin was particularly inefficient, because a sufficient concentration of wild-type pro-BDNF cannot be reached before the digestion step, and the wild-type pro-BDNF is not completely digested even after 48 hours of digestion.

## Claims

1. A human pro-BDNF mutein comprising the amino acid sequence set forth in SEQ ID NO: 8.

2. A nucleic acid encoding for the human pro-BDNF mutein according to claim 1.

3. A process of recombinantly producing human BDNF (rhBDNF), preferably a correctly folded, biologically active human BDNF (rhBDNF), from a human pro-BDNF mutein according to claim 1, in a prokaryotic expression system.

4. The process according to claim 3, said process comprising the steps of:
a) providing said human pro-BDNF mutein;
b) cleaving the pro-sequence of said human pro-BDNF mutein by a TEV protease, thus obtaining said rhBDNF, preferably said correctly folded, biologically active rhBDNF; and
c) isolating and purifying said rhBDNF, preferably said correctly folded, biologically active rhBDNF.

5. The process according to claim 4, wherein said step a) comprises the following steps:
i) expressing a human pro-BDNF mutein according to claim 1 in inclusion bodies;
ii) solubilizing said inclusion bodies in a denaturing solution, thereby obtaining a solution of denatured human pro-BDNF mutein;
iii) diluting the solution of denatured human pro-BDNF mutein into a refolding solution, thus obtaining a correctly folded human pro-BDNF mutein,
iv) purifying said correctly folded human pro-BDNF mutein from the solution provided in step iii).

6. The process according to claim 5, wherein said denaturing solution comprises:
- 1M - 8M, preferably 3M - 6M, Guanidinium-HCl;
- 0.05 M - 0.2 M, preferably 0.1 M, Tris buffer,
- 2.5 mM -10 mM, preferably 5 mM cysteine HCl; and
- 5 mM -15 mM, preferably 10mM, EDTA.

7. The process according to claim 5 or 6, wherein said refolding solution comprises:
- a chaperone, preferably Arginine,
- at least one metal chelator, preferably EDTA,
- a redox shuffling system, preferably Cystine and Cysteine-HCl,
said refolding solution having a pH comprised between 8.0 and 11.

8. The process according to claim 5 to 6, wherein said refolding solution comprises: 0.5-1 M Arginine, preferably Arginine-HCl, 2-10 mM EDTA, 2-10 mM Cysteine HCl, 0.5-2 mM Cystine, pH 8.5-10.

9. The process according to any one of claims 4 to 8, wherein step iv) of purifying said correctly folded human pro-BDNF mutein from the solution provided in step iii) is carried out via chromatography, preferably via mixed mode chromatography.

10. The process according to any one of claims 4 to 9, wherein said TEV protease is a wild-type TEV protease or a mutated TEV protease recognizing the recognition site sequence as set forth in SEQ ID NO: 10.

11. The process according to any one of claims 4 to 10, wherein in step b) the ratio of TEV protease to said pro-BDNF mutein is comprised between 1:2 and 1:500 w/w, preferably between 1: 10 and 1: 250 w/w, more preferably is a ratio of 1:50 w/w.

12. The process according to any one of claims 4 to 11, wherein step b) of cleaving is carried out for a time range comprised between 20 hours and 100 hours, preferably between 48 hours and 72 hours, at room temperature.

13. The process according to any one of claims 5 to 12, wherein said step i) comprises the steps of:
- providing a nucleic acid sequence encoding for a human pro-BDNF mutein according to claim 2;
- introducing said nucleic acid sequence into an expression vector, preferably a prokaryotic expression vector;
- introducing said expression vector into a prokaryotic cell;
- growing said prokaryotic cell in a suitable liquid culture medium, wherein said prokaryotic cell expresses a human pro-BDNF mutein according to claim 1, in form of inclusion bodies;
- lysing the prokaryotic cells and isolating said inclusion bodies.

14. A correctly folded, biologically active human BDNF (rhBDNF) obtained by the process according to any one of claims 4 to 13.

15. Use of a pro-BDNF mutein according to claim 1, for the preparation of a rhBDNF, preferably a correctly folded, biologically active rhBDNF.
